# EUROPEAN PATENT SPECIFICATION

(11) **EP 3 399 048 B1**
(45) Date of publication and mention of the grant of the patent: **09.11.2022**
(21) Application number: 16882128.8
(22) Date of filing: 29.12.2016
(51) Int. Cl.: C12Q 1/02, C12N 1/20, B60H 3/00, A61L 9/01, A61L 9/14

(54) **METHOD FOR SCREENING FOR ANTIMICROBIAL AGENT**
SCREENING-VERFAHREN FÜR ANTIMIKROBIELLES MITTEL
PROCÉDÉ DE CRIBLAGE POUR IDENTIFIER UN AGENT ANTIMICROBIEN

(30) Priority: 29.12.2015 KR 20150188643; 29.12.2015 KR 20150188631; 29.12.2015 KR 20150188680; 29.12.2015 KR 20150188675; 29.12.2015 KR 20150188667; 29.12.2015 KR 20150188657
(43) Date of publication of application: 07.11.2018
(73) Proprietor: Hyundai Motor Company, Seoul 06797 (KR); Kia Corporation, Seocho-gu Seoul 06797 (KR)
(72) Inventor: PARK, So Yoon, Gyeonggi-do, 18280 (KR); LEE, Tae Hee, Gyeonggi-do, 18280 (KR); KIM, Ji Wan, Gyeonggi-do, 18280 (KR); YOON, Ki Young, Gyeonggi-do, 18280 (KR)
(74) Representative: Isarpatent
(86) International application number: PCT/KR2016/015500
(87) International publication number: WO 2017/116176

(56) References cited:
- WO-A1-2015/088237
- JP-A- H1 199 193
- JP-A- 2008 523 820
- KR-A- 20050 064 705
- KR-A- 20150 067 562
- KR-A- 20150 067 563
- ROBERT B. SIMMONS ET AL: "The Occurrence and Persistence of Mixed Biofilms in Automobile Air Conditioning Systems", CURRENT MICROBIOLOGY, vol. 39, no. 3, 1 September 1999 (1999-09-01), pages 141-145, XP055260274, New York ISSN: 0343-8651, DOI: 10.1007/s002849900435
- LEE et al.: "Spirosoma Montaniterrae sp. nov., an Ultraviolet and Gamma Radiation- Resistant Bacterium Isolated from Mountain Soil", Journal of Microbiology, vol. 53, no. 7, July 2015 (2015-07), pages 429-434, XP055558444, DOI: 10.1007/s12275-015-5008-5

## Description

### [Technical Field]

The present disclosure relates to a method for screening for antimicrobial agents to control odor-causing microorganisms in an air conditioning system and a method for removing odors in an air conditioning system.

### [Background Art]

Clean air is recognized as essential to human health and well-being, and air inducing offensive odors or contaminated air is a main culprit that hinders the pleasant environment. For example, quality of unsatisfactory indoor air under closed conditions is caused by the following two important factors: one is indoor air contamination that is generated directly from the material (building, vehicle, etc.) itself constituting the enclosed environment (building, vehicle, etc.) and the another factor is the smell which is caused by human activity or a substance fed from the outside.

Air conditioning systems are systems that reduce the indoor temperature and optimize the indoor environment, for air conditioning including conditioning the temperature, humidity, airflow and cleanliness of the air in buildings, vehicles, trains, ships, aircraft, and the like. These air conditioning systems are becoming more popular with improvement in standards of living. However, although the prevalence of air conditioning systems has brought about a great development in basic functions, many environmental issues to improve the quality of indoor air remain unsolved.

Although the cause of odor of air conditioners among air conditioning systems is known to be metabolites of fungi and bacteria, there is no specific data regarding the types of fungi and bacteria, and amounts of metabolites secreted by the corresponding microorganisms.

Due to the structure of the air conditioning system, all air passing through the blower passes through the evaporator core (eva core). When heat exchange is carried out between cold refrigerant and air, condensate water condenses on the surface of the evaporator core due to temperature difference. The continuous condensation of condensate water provides an environment beneficial to propagation of fungi and bacteria. When fungi and bacteria proliferate in the evaporator core exposed to outside air, volatile organic compounds (mVOCs) of microorganisms are produced from metabolites of bacteria perforated on the surface of the evaporator core. When the air passing through the evaporator core is blown into the room, the room may be exposed to odor of fungi and bacteria upon use for a long time due to volatile organic compounds produced by microorganisms.

The surface of the evaporator core where odors are emitted is covered with a biofilm as the air conditioning system is used for a long period of time. Biofilms are composed of bacteria, cell clusters and EPS. EPS contains a variety of ingredients including proteins, polysaccharides, polyuronic acid, nucleic acids, lipids and the like. On the surface of the evaporator core, a variety of bacteria and fungi proliferate using biofilms as nutrients to release organic compounds (mVOCs) as metabolites by microorganisms. At this time, the odor emitted from the organic compounds (mVOCs) is known as rancidity among various factors of offensive odor of air conditioners.

Although various types of fragrances to remove offensive odors are commercially available, such fragrances cannot fundamentally remove fungi and bacteria growing on the evaporator core, and merely serve to temporarily relieve unpleasant odors. Currently commercially available antimicrobial agents are sold just because they have antibacterial activities to common pathogens, even though they have not been developed to target specific fungi or bacteria.

Accordingly, there is an urgent need to develop antimicrobial agents which are capable of creating a pleasant indoor air environment and technologies which are capable of removing unpleasant odors using the antimicrobial agents by clearly identifying the kinds of fungi and bacteria grown on the evaporator core and specifically blocking or preventing propagation of fungi and bacteria.

KR 2015 0067563 A and WO 2015/088237 A1 disclose examples of microorganisms that were found responsible for causing odors in air-conditioning systems.

The above description of the background art is intended only to improve understanding of the background of the present disclosure and should not be construed as recognizing that the above-described technologies are known to those having ordinary skill in the technical field to which the present disclosure pertains.

### [Disclosure]

### [Technical Problem]

The present inventors made attempts to find methods of effectively controlling odor-generating microorganisms using odorless microorganisms.

Therefore, the present disclosure has been made in view of the above problems, and it is one object of the present disclosure to provide a method for screening an antimicrobial agent against at least one microorganism causing an offensive odor in an air-conditioning system, wherein the microorganism is selected from the group consisting of *Pelomonas puraquae, Spirosoma radiotolerans, Fibrella aestuarina, Chryseobacterium geocarposphaerae, Spirosoma linguale,* and *Geobacillus toebii.*

It is another object of the present disclosure to provide a method for inhibiting growth of odor-causing microorganisms in an air-conditioning system including applying or spraying an antimicrobial agent onto the air-conditioning system.

It is another object of the present disclosure to provide a method for removing offensive odors in an air-conditioning system including isolating or removing odor-causing microorganisms from the air-conditioning system.

It is another object of the present disclosure to provide a method for removing offensive odors in an air-conditioning system including inhibiting growth of odor-causing microorganisms in the air-conditioning system.

Other features and aspects of the present disclosure will be apparent from the following detailed description, drawings and claims.

### [Technical Solution]

In one aspect, the present disclosure provides a method for screening for antimicrobial agents to reduce offensive odors in an air-conditioning system including the following steps:
To be specific, the method is a method for screening an antimicrobial agent in an air-conditioning system to remove odors generated from at least one microorganism selected from the group consisting of *Pelomonas puraquae, Spirosoma radiotolerans, Fibrella aestuarina*, *Chryseobacterium geocarposphaerae, Spirosoma linguale,* and *Geobacillus toebii*,
(a) preparing the microorganism or a culture solution thereof;
(b) bringing the microorganism or a culture solution thereof into contact with a sample;
(c) measuring growth inhibition of the microorganism; and
(d) considering the sample to have antibacterial activity to reduce offensive odors in an air-conditioning system, when growth of the microorganism is inhibited.

The present inventors made attempts to identify microorganisms causing offensive odors and found methods which are capable of effectively controlling microorganisms. As a result, they successfully isolated six species of microorganisms, which created and grew a biofilm in an air conditioning system, and found that offensive odors generated from the air conditioning system can be significantly prevented by controlling these microorganisms.

As herein used, the term "air conditioning system" generically refers to a system which can maintain the temperature, humidity, cleanliness, flow or the like of air pleasant in an area, a part or entirety of which is isolated from an outdoor environment. Preferably, for example, the isolated area may be an indoor area, a part or the entirety of which is isolated from an outdoor environment, like the inside of a building or the inside of a vehicle, train, ship, aircraft or the like. Preferably, the air conditioning system is for example an air conditioner.

Based on the structure of the air conditioning system, all air having passed through a blower passes through the evaporator core and condensate water continuously condenses on the surface of the evaporator core due to temperature difference, providing an environment which is beneficial for growth of microorganisms. After a long time, a biofilm is formed. At this time, microorganisms metabolize various indoor and outdoor materials as nutrients present in the air, generating odors derived from volatile organic compounds (mVOCs) produced as a result of metabolism.

Biofilms are a form of microbial communities wherein microorganisms live as clusters, have a structure in which a layer is surrounded by one membrane, and serve to protect microorganisms from the outside environment and provide nutrients. Exopolymeric substances (EPSs) are present as an ingredient constituting the film and contain a variety of ingredients such as proteins, polysaccharides, polyuronic acids, nucleic acids, and lipids. On the surface of the evaporator core, various microorganisms proliferate from the substances as nutrients and emit unpleasant odors from metabolites.

The present inventors isolated microorganisms which generate odors from the evaporator core and, as a result of culture of the microorganisms, separated and cultured dominant strains among microorganisms forming colonies. The method of separating and culturing dominant strains can be carried out using a variety of methods well-known to those skilled in the art. For example, dominant microorganisms can be selected through morphological approaches, such as dilution rate, and color, size or shape of colonies.

The dominant microorganism includes the genera *Fibrella*, *Chryseobacterium*, *Spirosoma*, *Geobacillus*, or *Pelomonas,* preferably, *Fibrella aestuarina, Chryseobacterium geocarposphaerae, Spirosoma linguale, Spirosoma radiotolerans*, *Geobacillus toebii*, or *Pelomonas puraquae.*

The microorganisms were deposited at the Korea Culture Center of Microorganisms on April 17, 2015 and were given the following accession numbers: *Fibrella aestuarina* HKMC-115 (accession number: KCCM 11691P), *Chryseobacterium geocarposphaerae* HKMC-116 (accession number: KCCM 11692P), *Spirosoma linguale* HKMC-117 (accession number: KCCM 11693P), *Spirosoma radiotolerans* HKMC-114 (accession number: KCCM 11690P), *Geobacillus toebii* HKMC-118 (accession number: KCCM 11694P), and *Pelomonas puraquae* HKMC-113 (accession number: KCCM 11689P) .

The odor-causing microorganisms have a variety of industrial applicability. For example, the odor-causing microorganisms can be used to develop novel antibacterial agents to inhibit growth of microorganisms and develop an air freshener for removing offensive odors by identifying the chemical properties of the metabolites of the microorganisms. In addition, the odor-causing microorganisms may be used to fundamentally remove the cause of offensive odors by providing an air-conditioning system with an environment where the microorganisms cannot live.

The sample used in the method for screening an antimicrobial agent of the present disclosure is used to determine whether it has antimicrobial activity against the microorganisms. For example, when a particular sample has antimicrobial activity against *Pelomonas puraquae,* the sample may be screened as an antimicrobial agent against *Pelomonas puraquae.*

Preferably, the antimicrobial agent screened by the screening method of the present disclosure may have antimicrobial activity against *Pelomonas puraquae,* more preferably, against other species of microorganisms.

For example, some antimicrobial agents may have antimicrobial activity against all six species of microorganisms and another antimicrobial agent may have no antimicrobial activity at all against one or more of the species. In addition, the antimicrobial agent having antimicrobial activity against all six species of microorganisms may have different antimicrobial activity against different microorganisms (see Table 8).

In one preferred exemplary embodiment of the present disclosure, the sample to be screened includes a single compound, a mixture of compounds, an animal or plant extract, a biological agent containing genetic information such as a nucleotide, a polypeptide and the like, and a mixture of compound and biological agent.

Preferably, the microorganism causing an offensive odor includes one or more selected from the group consisting of *Pelomonas puraquae*, *Spirosoma radiotolerans*, *Fibrella aestuarina*, *Chryseobacterium geocarposphaerae*, *Spirosoma linguale*, and *Geobacillus toebii.*

In another aspect, the present disclosure provides a method for inhibiting the growth of a microorganism causing offensive odors in an air-conditioning system, including coating or spraying the antimicrobial agent onto an air-conditioning system.

The antimicrobial agent that may be used in the present disclosure may be any antimicrobial agent which is determined or can be determined to have antimicrobial activity against one or more microorganisms selected from the group consisting of *Pelomonas puraquae, Spirosoma radiotolerans, Fibrella aestuarina, Chryseobacterium geocarposphaerae, Spirosoma linguale,* and *Geobacillus toebii*.

The antimicrobial agent may be coated or sprayed into an air-conditioning system to inhibit growth of the odor-causing microorganisms and microorganisms including the same, and the coating or spraying may be carried out in various forms known in the art, such as gas, liquid, gel or suspension of a solid.

In addition, the coating or spraying may be performed partly or wholly on the inner surface or inner components of the air-conditioning system. Preferably, the coating or spraying may be performed on an evaporator core in the air-conditioning system. The inhibition of growth can be carried out by applying or spraying the antimicrobial agent after the odor-causing microorganisms form a biofilm or by applying or spraying antimicrobial agent to prevent growth of the microorganisms before the odor-causing microorganisms form a biofilm.

In another aspect, the present disclosure provides a method for removing an offensive odor in an air-conditioning system including isolating or removing a microorganism causing offensive odors from the air-conditioning system.

The removal of offensive odors includes all or some of offensive odors, and the coating or spraying may be performed to prevent offensive odors before the offensive odors are generated.

Various microorganisms proliferate in an air-conditioning system. These microorganisms can be broadly classified into microorganisms causing offensive odors and microorganisms not causing offensive odors. Accordingly, when the antimicrobial agent acts specifically only on the microorganisms causing offensive odors or has inhibitory activity against the growth of all or some of the dominant species of microorganisms causing offensive odors, the offensive odors of the air-conditioning system may be partially or completely removed or improved.

In another aspect, the present disclosure provides a method for removing offensive odors in an air-conditioning system, including isolating or removing microorganisms causing offensive odors from an air-conditioning system.

The microorganisms described above or microorganisms including the same may be partially or completely isolated or removed via a physical, chemical or biological method. The physical method may be one of artificially isolating or removing the aforementioned microorganism or a microorganism including at least one of the same using a physical apparatus. The chemical method may be one of isolating or removing the aforementioned microorganism or a microorganism including at least one of the same using an antimicrobial agent or a sterilizer against the microorganism. The biological method may be one of isolating or removing the microorganisms using a biological agent which is toxic to the microorganisms or using another microorganism which competes with the microorganism for survival. However, the present disclosure is not limited by these examples.

In another aspect, the present disclosure provides a method for removing offensive odors in an air conditioning system including inhibiting growth of odor-causing microorganisms in the air conditioning system.

### [Advantageous effects]

The present disclosure provides a microorganism causing offensive odors in an air-conditioning system. In addition, the present disclosure provides a method for screening for an antibacterial agent to control microorganisms. Furthermore, the present disclosure provides a method for removing offensive odors in an air-conditioning system by controlling the microorganisms.

The odor-causing microorganisms in the air-conditioning system can be used to develop novel antibacterial agents or to develop an air fresher to block offensive odors by identifying the chemical properties of metabolites of microorganisms. In addition, the odor-causing microorganisms have various industrial applicability of fundamentally removing the cause of odors by previously creating an environment to prevent growth of the microorganisms in the air-conditioning system.

### [Description of Drawings]

FIG. 1 is an image showing a specimen sampled from an evaporator core in an odor-causing secondhand vehicle;
FIG. 2 is an image showing a method of testing antibacterial activity according to the present disclosure; and
FIG. 3 is an image showing culturing combinations of dominant odorless microorganisms using an aluminum fin which is a material for an evaporator core.

### [Best Mode]

Hereinafter, the present disclosure will be described in more detail with reference to examples. These examples are provided only for illustration of the present disclosure and it would be obvious to those skilled in the art that the scope of the present disclosure is not limited by the examples depending on the subject matter of the present disclosure.

### Example 1: Selection of odor-causing dominant microorganisms

### 1. Acquisition of odorous vehicles and separation of air-conditioning system

In order to identify the cause of offensive odors generated in a closed environment, like the inside of a vehicle, the present inventors secured ten kinds of second-hand vehicles generating offensive odors depending on season (winter: February to March, summer: June to July), isolated an air-conditioning system mounted on the respective vehicles, and detached an evaporator core where a biofilm is predicted to be formed by odor-causing microorganisms to conduct specimen sampling (Table 1).

**[TABLE 1]**

| No. | Vehicle mileage | Season |
|---|---|---|
| 1 | 89,000 km | Winter (Feb-Mar) |
| 2 | 70,000 km | |
| 3 | 10,300 km | |
| 4 | 37,100 km | |
| 5 | 149,970 km | |
| 6 | 35,000 km | Summer (Jun-Jul) |
| 7 | 28,000 km | |
| 8 | 42,000 km | |
| 9 | 110,000 km | |
| 10 | 90,000 km | |

### Example 2: Evaporator core specimen sampling

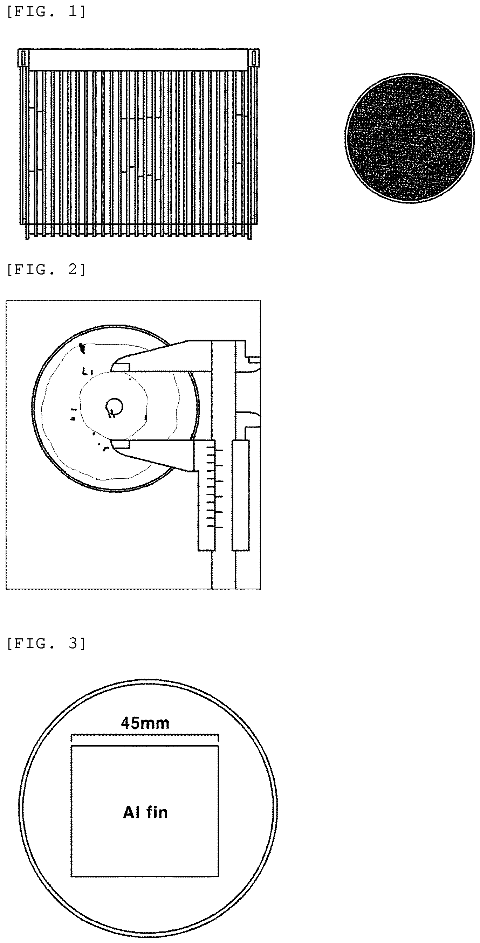

The evaporator core samples acquired from odorous second-hand vehicles 1 to 10 were sealed in a polyethylene bag and refrigerated at 4°C before use. In order to isolate and culture microorganisms, 5 g of specimens were collected from any spots including front and back parts in respective evaporator cores using sterilized long nose pliers and then mixed before use (FIG. 1).

### Example 3: Separation of microorganisms

The microorganisms were separated from specimens acquired from the evaporator cores in accordance with the following process.
① Specimens extracted from the evaporator cores were mixed and fed into a stirrer.
② Sterilized 1×phosphate buffed saline (PBS) was fed into a 200 ml stirrer.
③ The mixed specimen was stirred with PBS for 30 seconds.
④ The stirrer was placed on ice for one minute.
⑤ Steps ③ and ④ were repeated twice.
⑥ The suspension was centrifuged at 4°C and 13,000 rpm for 3 minutes.
⑦ Only the supernatant was collected and transferred to a new tube.
⑧ The sterilized swab was soaked with the supernatant and the surface of the evaporator core, from which the sample was collected, was cleaned with the swab several times.
⑨ Only the head of the cleaned swab was immersed in the supernatant and vortexing was conducted.
⑩ The precipitate obtained in step ⑥was mixed with the mixture of step ⑨and the resulting mixture was used as an inoculation stock.

After steps ① to ⑩, microorganisms were separated by physical detachment from evaporator cores mounted on vehicle models 1 to 10.

### Example 4: Separation of odor-causing microorganisms and selection of dominant strains

The separation of bacteria from the air conditioner is generally carried out by performing heterotrophic plate culture on aerobic heterotrophic bacteria which are called general bacteria. Separation of bacteria is carried out at 28 to 30°C for 14 days using two complex nutrient media including PTYG agar medium and R2A agar medium. In the case of PTYG agar medium, peptone 0.25 g (Difco), triptone 0.25 g (Difco), yeast extract 0.5 g (Difco), glucose 0.5 g (Difco), MgSO₄ 30 mg (Sigma), CaCl₂ 3 mg (Sigma), and Bactoagar 15g (Difco) were added to 980 ml of distilled water, pH was adjusted to 7.0 and the resulting mixture was autoclaved at 121°C for 15 minutes. In the case of R2A agar medium, yeast extract 0.5 g (Difco), proteose peptone No. 3 0.5 g (Difco), casamino acids 0.5 g (Difco), dextrose 0.5 g (Difco), soluble starch 0.5 g (Difco), sodium pyruvate 0.3 g (Difco), dipotassium sulfate 0.3 g (Difco), magnesium sulfate 0.05 g (Difco) and bacto agar 15 g (Difco) were added to distilled water 980 ml, pH was adjusted to 7.2 (final: 1,000 ml) and the resulting mixture was autoclaved at 121°C for 15 minutes. In order to isolate non-dominant bacteria, kanamycin, ampicillin and chloramphenicol were added at a concentration of 100 ppm when the medium temperature reached 50°C after sterilization of the filter, to produce antibiotic media.

To separately culture dominant strains, first, various dominant strains should be selected through morphological approach of dilution ratio, and color, size and shape of colonies and the like.
① Fungi and bacteria were separately isolated from the separately cultured media.
② A variety of bacteria having different morphologies were inoculated into a complex medium using a loop and purely isolated.
③ The medium growing the best was selected from inoculated media and passage culture was conducted.
④ The end of the mycelium of fungi was isolated with a scalpel and inoculated into a complex medium.
⑤ In the case of fungi strains as well, the medium growing the most was selected from inoculated media and passage culture was conducted.

### 5. Identification of dominant strains

In order to accurately identify the isolated microorganisms, 16s rRNA identification including the following steps was conducted.

### a) Investigation of fingerprints through REP-PCR pattern analysis

REP-PCR is a molecular biological method of analyzing the structure of bacterial chromosomes and is a fingerprinting method which is capable of distinguishing specific bacterial strains from other bacteria. Genetic characteristics were analyzed in accordance with respective processes to conduct REP-PCR.

### (1) Cell lysis process

① 2.5 µl of Lyse-N-Go PCR Reagent (Thermo) was placed in a PCR tube.
② Colonies were harvested with a pipette at a clean bench, placed in the tube and pipetting was conducted. The amount of colonies harvested should be determined not to make the solution slightly hazy.
③ According to manufacturer's instructions, culture was conducted in a PCR machine.

### (2) PCR reaction

Suitable amounts of ingredients required for PCR reaction described in the following Table 2 were mixed to prepare a reaction mixture and, as shown in Table 3, pre-denaturation at 93°C for 7 minutes, denaturation at 92°C for 1 minute, annealing at 51.5°C for 1 minute, and extension at 65°C for 8 minutes were conducted, and denaturation, annealing and extension processes were repeated 33 times to conduct PCR amplification.

**[Table 2]**

| | |
|---|---|
| ①dNTP (2.5 mM each) | 12.5 µl |
| ②Gitschier buffer | 5.0 µl |
| ③DMSO (100%) | 2.5 µl |
| ④Autoclaved 3°D.W. | 0.3 µl |
| ⑤BOXA1R primer(50 pmole/µl) 1.0 µl 5'CTACGGCAAGGCGACGCTGACG | |
| ⑥BSA (10 mg/ml) | 0.4 µl |
| ⑦Bacterial DNA | 2.5 µl |
| ⑧Taq polymerase (Roche) (5 U/µl) | 0.8 µl |

**[TABLE 3]**

| | | |
|---|---|---|
| step 1 | 193°C | 7 min |
| step 2 | 92°C | 1 min |
| step 3 | 51.5°C | 1 min |
| step 4 | 65°C | 8 min |
| step 2,3,4: additional 33 cycles | | |
| step 6 | 65°C | 16 min |
| step 7 | 4°C | |

### (3) Gel electrophoresis

The DNA fragments amplified by PCR were collected, 1.2-1.5% agarose gel supplemented with EtBr was used, and a mixture of 6x dye and a sample in a ratio of 1 to 5 was loaded in an amount as much as possible. Since most PCR products were between 100 and 1,000 bp, they were loaded with a 100 bp ladder, and electrophoresis was conducted as slow as possible such that the middle (50 V) of bromophenol blue and xylene cyanol dyes reached the middle of the entire gel. Strains that have identical DNA patterns on gel are considered to be the same strains.

### (b) Identification of air conditioner dominant bacteria through 16S rRNA genetic analysis

16S rRNA (ribosomal ribonucleic acid) genes are used for identification of genetic classes of bacteria and can be identified at the level of genus and species of bacteria classified by REP-PCR.

### (1) Cell lysis process

① 5 µl of Lyse-N-Go PCR Reagent (Thermo) was placed in a PCR tube.
② Colonies were harvested with a pipette at a clean bench, placed in the tube and pipetting was conducted. The amount of colonies harvested should be determined not to make the solution slightly hazy.
③ According to manufacturer's instructions, culture was conducted in a PCR machine (Table 4).

**[Table 4]**

| Cycle | Temperature(°C) | Time(seconds) |
|---|---|---|
| 1 | 65 | 30 |
| 2 | 8 | 30 |
| 3 | 65 | 90 |
| 4 | 97 | 180 |
| 5 | 8 | 60 |
| 6 | 65 | 180 |

### (2) 16S rRNA genetic PCR

PCR conditions (Total 50 µl): ingredients for the solution excluding DNAs and Taq were mixed in predetermined amounts as shown in the following Table 5 and the resulting mixture was added to 44.5 µl of a lysis solution. Then, as shown in the following Table 6, pre-denaturation at 94°C for 5 minutes, denaturation at 94°C for 1 minute, annealing at 55°C for 1 minute, and extension at 72°C for 1 min 30 seconds were conducted, and denaturation, annealing and extension steps were conducted 29 times to perform PCR amplification.

**[Table 5]**

| | |
|---|---|
| Autoclaved 3°D.W. | 22 µl |
| 10xbuffer (Roche) | 5 µl |
| dNTP (Roche, 2.5 mM) | 5 µl |
| DMSO | 5 µl |
| BSA (10 mg/ml) | 2.5 µl |
| 27mf (20 pmole/µl) | 2.5 µl |
| 1492r (20 pmole/µl) | 2.5 µl |
| DNA | 5 µl |
| Taq (Roche) | 0.5 µl |

**[Table 6]**

| | | |
|---|---|---|
| step 1 | 94°C | 5 min |
| step 2 | 94°C | 1 min |
| step 3 | 55°C | 1 min |
| step 4 | 72°C | 1 min 30 sec |
| Go to step 2: additional 29 cycles | | |
| step 6 | 72°C | 10 min |
| step 7 | 4°C | hold |

### (3) PCR purification

The products amplified by 16S-rRNA genetic PCR were purified using a Qiaquick PCR purification kit in accordance with the following process.
① 5x volume of PB buffer of the PCR product was added.
② The mixed solution was seeded on a QIAquick column.
③ For binding of DNAs, centrifugation was conducted for one minute and the supernatant was removed.
④ For washing, 750 µl of PE buffer was placed in a QIAquick column, centrifugation was conducted for one minute and the supernatant was removed.
⑤ Centrifugation was conducted again for one minute.
⑥ The QIAquick column was transferred to a new tube.
⑦ In order to extract DNAs, 30 µl of EB buffer was added thereto and was allowed to stand for one minute.
⑧ Centrifugation was conducted for one minute to allow DNAs dissolved in EB to be collected in the tube.

As a result of the test, in order to check whether or not purely isolated microorganisms generate odors, purely isolated microorganisms were cultured by the following method and sensory evaluation was conducted.
① Pure separately cultured microorganisms were inoculated into a liquid nutrient medium.
② The inoculated medium was cultured at 28°C for 5 to 7 days.
③ 100 µl of the bacteria cultured in the liquid medium was inoculated into a solid nutrient medium.
④ The inoculated bacteria was evenly spread using a spreader.
⑤ A petri dish was sealed and cultured at 28°C for 10 days.

Sensory evaluation was conducted, based on a 5-grade method using seven panels, odor-causing microorganisms were selected using the average after evaluation of odor intensity, six dominant strains were identified through identification of the 16S rRNA genetic analysis, and these dominant strains were deposited at the Korea Culture Center of Microorganisms on April 17, 2015.

**[Table 7]**

| No. | Identificatio n No. | Name of microorganism | Accessio n No. |
|---|---|---|---|
| 1 | HKMC-113 | *Pelomonas puraquae* | **KCCM 11689P** |
| 2 | HKMC-114 | *Spirosoma radiotolerans* | **KCCM 11690P** |
| 3 | HKMC-115 | *Fibrella aestuarina* | **KCCM 11691P** |
| 4 | HKMC-116 | *Chryseobacterium geocarposphaerae* | **KCCM 11692P** |
| 5 | HKMC-117 | *Spirosoma linguale* | **KCCM 11693P** |
| 6 | HKMC-118 | *Geobacillus toebii* | **KCCM 11694P** |

### Example 2: Evaluation of antibacterial activity of selected odor-causing microorganisms depending on antibacterial agent

### 1. Test process

The present inventors evaluated antibacterial activity of dominant microorganisms selected in Example 1 using a variety of commercially available antibacterial agents. The antibacterial agents used in the present disclosure will be given below:

Antibacterial agent A: Kimcare (Yuhan Kimberly, Ltd.)

Antibacterial agent B: Febreze (P&G)

Antibacterial agent C: mass-produced antibacterial agent containing methyl alcohol 45~50%, chromium sulfate (CAS 10101-53-8) 1~5%, bromine 1~5% and water

The evaluation of antibacterial activity was conducted by the following steps:
① Preparing sterilized filter paper
② Preparing three kinds of antibacterial agents (control group: group not-treated with antibacterial agent, test groups: antibacterial agent A, antibacterial agent B, antibacterial agent C)
③ Feeding filter paper to antibacterial agent
④ coating respective odor-causing microorganisms in a nutrient medium
⑤ placing antibacterial agent-containing filter papers in odor-causing microorganisms-coated nutrient medium
⑥ Culturing at 28 to 30°C for 5 days
⑦ Measuring an area of growth inhibition.

Measurement of the area of growth inhibition was carried out by measuring the diameter of the area of growth inhibition using Vernier calipers, and the method will be shown in detail in FIG. 2.

### 2. Test result

An average of three diameters of the area of growth inhibition obtained by testing each of six strains of odor-causing microorganisms three times is shown in Table 8.

**[Table 8]**

| **No.** | **Name of microorganism (Deposition name)** | **No antibacterial Agent** | **A** | **B** | **C** |
|---|---|---|---|---|---|
| 1 | *Pelomonas puraquae* HKMC-113 | 0 | 1.17 | 2.07 | 3.93 |
| 2 | *Spirosoma radiotolerans* HKMC-114 | 0 | 2.03 | 2.17 | 4.57 |
| 3 | *Fibrella aestuarina* HKMC-115 | 0 | 2.13 | 2.63 | 4.30 |
| 4 | *Chryseobacterium geocarposphaerae* HKMC-116 | 0 | 0.97 | 1.57 | 2.70 |
| 5 | *Spirosoma linguale* HKMC-117 | 0 | 1.83 | 2.10 | 4.83 |
| 6 | *Geobacillus toebii strain* R-35642HKMC-118 | 0 | 1.73 | 1.93 | 2.20 |

| | | | | | |
|---|---|---|---|---|---|
| **(unit: cm)** | | | | | |

As shown in Table 8, antibacterial agent A exhibits weaker antibacterial activity against *Chryseobacterium geocarposphaerae,* and antibacterial agent B exhibits strong antibacterial activity against *Fibrella aestuarina,* but weaker antibacterial activity against *Chryseobacterium geocarposphaerae.*

In addition, antibacterial agent C exhibits weak antibacterial activity against *Geobacillus toebii*, but exhibits stronger overall antibacterial activity against six strains of microorganisms than antibacterial agents A and B.

### Example 3: Evaluation of odors of evaporator core from which odor-causing microorganisms are removed.

In order to reproduce the evaporator core from which odorous microorganisms were removed or separated, the present inventors cultured a combination of odorless microorganisms excluding the odorous microorganism of Example 1, among dominant microorganisms grown in the evaporator core, using an aluminum fin which is a material for the evaporator core (Table 9, FIG. 3).

Odorless microorganisms were selected as dominant microorganisms which were grown in the evaporator core, created colonies during culture and did not generate an offensive odor and the culture method will be given below:
① Purely isolated and cultured odorless microorganisms were inoculated in R2A liquid medium.
② The inoculated medium was cultured at 28°C for 5 to 7 days.
③ An aluminum fin which had been sterilized at a high pressure at 121°C for 20 minutes was prepared.
④ The fin was immersed in each antibacterial agent to evenly coat the surface of the fin.
⑤ The coated aluminum fin was placed on a petri dish.
⑥ 1 ml of the cultured odorless microorganism inoculation solution was centrifuged and the supernatant was removed.
⑦ 1 ml of the sterilized 1 × PBS was added and centrifugation was conducted again.
⑧ The method of ⑦ was repeated twice.
⑨ 100 µl of the odorless microorganism washed with PBS 100 µl was dropped in the middle of the aluminum fin.
⑩ The prepared aluminum fin was inoculated with microorganisms and dried at room temperature.
⑪ The petri dish was sealed and at 28°C for one month.

As a result, all the combinations of the following table 9 (result of odor analysis upon culture of microorganisms grown in evaporator core from which odorous microorganisms are removed) did not generate odors after one month.

**[Table 9]**

| **Combination** | **Microorganism** | **Odor evaluation after one month** |
|---|---|---|
| 1 | *Methylobacterium brachiatum* | odorless |
| 2 | *Methylobacterium platani* | odorless |
| 3 | *Methylobacterium aquaticum* + *Methylobacterium platani* | odorless |
| 4 | *Methylobacterium platani* + *Methylobacterium brachiatum* | odorless |
| 5 | *Methylobacterium aquaticum* + *Methylobacterium platani* + *Methylobacterium brachiatum* | odorless |
| 6 | *Methylobacterium aquaticum* + *Methylobacterium platani* + *Methylobacterium brachiatum* + *Acinetobacter johnsonii* | odorless |
| 7 | *Methylobacterium aquaticum* + *Methylobacterium platani* + *Methylobacterium brachiatum* + *Bacillus vietnamensis* | odorless |
| 8 | *Methylobacterium aquaticum* + *Methylobacterium platani* + *Methylobacterium brachiatum* + *Brevibacillus invocatus* | odorless |
| 9 | *Methylobacterium aquaticum* + *Methylobacterium platani* + *Methylobacterium brachiatum* + *Deinococcus ficus* | odorless |
| 10 | *Methylobacterium aquaticum* + *Methylobacterium platani* + *Methylobacterium brachiatum* + *Leifsonia soli* | odorless |
| 11 | *Methylobacterium aquaticum* + *Methylobacterium platani* + *Methylobacterium brachiatum* + *Methylobacterium komagatae* | odorless |
| 12 | *Methylobacterium aquaticum* + *Methylobacterium platani* + *Methylobacterium brachiatum* + *Pseudomonas nitroreducens* | odorless |
| 13 | *Methylobacterium aquaticum* + *Methylobacterium platani* + *Methylobacterium brachiatum* + *Sphingomonas aquatilis* | odorless |
| 14 | *Sphingomonas aquatilis* + *Brevibacillus invocatus* | odorless |
| 15 | *Leifsonia soli* + *Methylobacterium komagatae* | odorless |
| 16 | *Acinetobacter johnsonii* + *Sphingomonas aquatilis* + *Methylobacterium komagatae* | odorless |
| 17 | *Pseudomonas nitroreducens* | odorless |
| 18 | *Acinetobacter johnsonii* + *Pseudomonas nitroreducens* | odorless |
| 19 | *Brevibacillus invocatus* + *Acinetobacter johnsonii* + *Pseudomonas nitroreducens* | odorless |
| 20 | *Leifsonia soli* + *Pseudomonas nitroreducens* | odorless |
| 21 | *Brevibacillus invocatus* + *Sphingomonas aquatilis* + *Pseudomonas nitroreducens* | odorless |
| 22 | *Acinetobacter johnsonii* + *Sphingomonas aquatilis* + *Pseudomonas nitroreducens* | odorless |
| 23 | *Methylobacterium aquaticum* + *Methylobacterium komagata e* + *Bacillus vietnamensis* + *Deinococcus ficus* | odorless |
| 24 | *Methylobacterium aquaticum* + *Methylobacterium komagata e* + *Curtobacterium flaccumfaciens* + *Deinococcus apachensis* + *Bacillus subtilis subsp. subtilis* | odorless |
| 25 | *Methylobacterium aquaticum* + *Methylobacterium komagata e* + *Spirosoma linguale* + *Sphingomonas dokdonensis* + *Leifsoniasoli* | odorless |

As shown in the test result described above, when a combination of microorganisms not generating odors is formed by removing odor-causing microorganisms grown in an air-conditioning system by a chemical or physical method, odors generated from the air-conditioning system can be significantly removed.

## Claims

1. A method for screening for an antimicrobial agent in an air-conditioning system to remove odors generated in the air-conditioning system from at least one microorganism selected from the group consisting of *Pelomonas puraquae, Spirosoma radiotolerans, Fibrella aestuarina, Chryseobacterium geocarposphaerae*, *Spirosoma linguale*, and *Geobacillus toebii*, the method comprising: (a) preparing the microorganism or a culture solution thereof;
(b) bringing the microorganism or a culture solution thereof into contact with a sample;
(c) measuring growth inhibition of the microorganism; and
(d) considering the sample to have antibacterial activity to reduce odors in an air-conditioning system, when growth of the microorganism is inhibited.

2. The method according to claim 1, wherein the air-conditioning system is an air conditioner.

3. The method according to claim 1, wherein the microorganism forms a biofilm in the evaporator core in the air-conditioning system to induce odors.

4. The method according to claim 3, wherein a material for the evaporator core is aluminum, an aluminum alloy, copper or a copper alloy.

5. The method according to claim 1, wherein the *Pelomonas puraquae* is *Pelomonas puraquae* HKMC-113 (accession number: KCCM11689P), the *Spirosoma radiotolerans* is *Spirosoma radiotolerans* HKMC-114 (accession number: KCCM11690P), the *Fibrella aestuarina is Fibrella aestuarina* HKMC-115 (accession number: KCCM11691P), the *Chryseobacterium geocarposphaerae* is *Chryseobacterium geocarposphaerae* HKMC-116 (accession number: KCCM11692P), the *Spirosoma linguale* is *Spirosoma linguale* HKMC-117 (accession number: KCCM11693P), and the *Geobacillus toebii* is *Geobacillus toebii* HKMC-118 (accession number: KCCM11694P).

6. A method for removing odors in an air-conditioning system comprising, from the air-conditioning system, isolating or removing, or inhibiting growth of, at least one odor-causing microorganism selected from the group consisting of *Pelomonas puraquae*, *Spirosoma radiotolerans*, *Fibrella aestuarina, Chryseobacterium geocarposphaerae, Spirosoma linguale,* and *Geobacillus toebii*.

## Patentansprüche

1. Screening-Verfahren für ein antimikrobielles Mittel in einem Luftkonditionierungssystem, um in dem Luftkonditionierungssystem erzeugte Gerüche von mindestens einem Mikroorganismus zu beseitigen, der aus der Gruppe ausgewählt ist, die aus *Pelomonas puraquae*, *Spirosoma radiotolerans*, *Fibrella aestuarina, Chryseobacterium geocarposphaerae*, *Spirosoma linguale* und *Geobacillus toebii* besteht, wobei das Verfahren umfasst:
(a) Präparieren des Mikroorganismus oder einer Kulturlösung des Mikroorganismus;
(b) Inkontaktbringen des Mikroorganismus oder einer Kulturlösung des Mikroorganismus mit einer Probe;
(c) Messen der Wachstumshemmung des Mikroorganismus; und
(d) Bestimmen, dass die Probe antibakterielle Aktivität besitzt, um Gerüche in einem Luftkonditionierungssystem zu reduzieren, wenn das Wachstum des Mikroorganismus gehemmt wird.

2. Verfahren nach Anspruch 1, wobei das Luftkonditionierungssystem eine Klimaanlage ist.

3. Verfahren nach Anspruch 1, wobei der Mikroorganismus einen Biofilm im Verdampferkern des Luftkonditionierungssystems bildet, um Gerüche zu induzieren.

4. Verfahren nach Anspruch 3, wobei das Material für den Verdampferkern Aluminium, eine Aluminiumlegierung, Kupfer oder eine Kupferlegierung ist.

5. Verfahren nach Anspruch 1, wobei es sich bei *Pelomonas puraquae* um *Pelomonas puraquae* HKMC-113 (Hinterlegungsnummer: KCCM11689P) handelt, bei *Spirosoma radiotolerans* um *Spirosoma radiotolerans* HKMC-114 (Hinterlegungsnummer: KCCM11690P) handelt, bei *Fibrella aestuarina* um *Fibrella aestuarina* HKMC-115 (Hinterlegungsnummer: KCCM11691P) handelt, bei *Chryseobacterium geocarposphaerae* um *Chryseobacterium geocarposphaerae* HKMC-116 (Hinterlegungsnummer: KCCM11692P) handelt, bei *Spirosoma linguale* um *Spirosoma linguale* HKMC-117 (Hinterlegungsnummer: KCCM11693P) handelt, und bei *Geobacillus toebii* um *Geobacillus toebii* HKMC-118 (Hinterlegungsnummer: KCCM11694P) handelt.

6. Verfahren zum Beseitigen von Gerüchen in einem Luftkonditionierungssystem, umfassend, aus dem Luftkonditionierungssystem mindestens einen geruchsverursachenden Mikroorganismus, der aus der Gruppe ausgewählt ist, die aus *Pelomonas puraquae*, *Spirosoma radiotolerans, Fibrella aestuarina, Chryseobacterium geocarposphaerae*, *Spirosoma linguale* und *Geobacillus toebii* besteht, zu isolieren oder zu beseitigen oder dessen Wachstum zu hemmen.

## Revendications

1. Procédé de criblage pour identifier un agent antimicrobien dans un système de conditionnement d'air pour éliminer les odeurs générées dans le système de conditionnement d'air à partir d'au moins un microorganisme choisi dans le groupe consistant en *Pelomonas puraquae*, *Spirosoma radiotolerans*, *Fibrella aestuarina, Chryseobacterium geocarposphaerae, Spirosoma linguale* et *Geobacillus toebii,* le procédé comprenant :
(a) la préparation du microorganisme ou d'une solution de culture de celui-ci ;
(b) la mise en contact du microorganisme ou d'une solution de culture de celui-ci avec un échantillon ;
(c) la mesure de l'inhibition de croissance du microorganisme ;
et
(d) la considération du fait que l'échantillon a une activité antibactérienne pour réduire les odeurs dans un système de conditionnement d'air, lorsque la croissance du microorganisme est inhibée.

2. Procédé selon la revendication 1, dans lequel le système de conditionnement d'air est un climatiseur.

3. Procédé selon la revendication 1, dans lequel le microorganisme forme un biofilm dans le noyau de l'évaporateur dans le système de conditionnement d'air pour induire des odeurs.

4. Procédé selon la revendication 3, dans lequel un matériau pour le noyau de l'évaporateur est l'aluminium, un alliage d'aluminium, le cuivre ou un alliage de cuivre.

5. Procédé selon la revendication 1, dans lequel le *Pelomonas puraquae* est *Pelomonas puraquae* HKMC-113 (numéro d'ordre : KCCM11689P), le *Spirosoma radiotolerans* est *Spirosoma radiotolerans* HKMC-114 (numéro d'ordre : KCCM11690P), le *Fibrella aestuarina* est *Fibrella aestuarina* HKMC-115 (numéro d'ordre : KCCM11691P), le *Chryseobacterium geocarposphaerae* est *Chryseobacterium geocarposphaerae* HKMC-116 (numéro d'ordre : KCCM11692P), le *Spirosoma linguale* est *Spirosoma linguale* HKMC-117 (numéro d'ordre : KCCM11693P), et le *Geobacillus toebii* est *Geobacillus toebii* HKMC-118 (numéro d'ordre : KCCM11694P).

6. Procédé pour éliminer les odeurs dans un système de conditionnement d'air comprenant, à partir du système de conditionnement d'air, l'isolement ou l'élimination, ou l'inhibition de la croissance, d'au moins un microorganisme provoquant des odeurs choisi dans le groupe consistant en *Pelomonas puraquae, Spirosoma radiotolerans, Fibrella aestuarina, Chryseobacterium geocarposphaerae, Spirosoma linguale* et *Geobacillus toebii*.
